# EUROPEAN PATENT SPECIFICATION

(11) **EP 1 586 553 B1**
(45) Date of publication and mention of the grant of the patent: **22.04.2009**
(21) Application number: 05006414.6
(22) Date of filing: 23.03.2005
(51) Int. Cl.: C07C 209/68, C07C 213/08, C07D 207/12

(54) **Process for preparing amines**
Verfahren zur Herstellung von Aminen
Procédé pour la préparation des amines

(30) Priority: 30.03.2004 JP 2004097461
(43) Date of publication of application: 19.10.2005
(73) Proprietor: DAISO CO., LTD., Osaka-shi Osaka-fu (JP)
(72) Inventor: Yaegashi, Keisuke, Osaka-shi Osaka-fu (JP); Mikami, Masafumi, Osaka-shi Osaka-fu (JP)
(74) Representative: Vossius & Partner

(56) References cited:
- GB-A- 924 042
- MEHLER, T. ET AL: "Enantioselective addition of diethylzinc to aromatic aldehydes catalyzed by chiral ligands derived from L-hydroxyproline" SYNTHETIC COMMUNICATIONS , 23(19), 2691-9 CODEN: SYNCAV; ISSN: 0039-7911, 1993, XP009033906
- HASHIMOTO, M.; EDA, Y.; OSANAI, Y. ET AL.: "A novel decarboxylation of alpha-amino acids. A facile method of decarboxylation by the use of 2-cyclohexen-1-one as a catalyst" CHEMISTRY LETTERS, 1986, pages 893-896, XP000891448
- WALLBAUM, SABINE ET AL: "Decarboxylation of .alpha.-amino acids containing two and three stereogenic centers: a simple one-step procedure to prepare two optically active .beta.-amino alcohols and a bicyclic pyrrolidine derivative" SYNTHETIC COMMUNICATIONS , 24(10), 1381-7 CODEN: SYNCAV; ISSN: 0039-7911, 1994, XP009050459
- PANDEY, G. AND CHAKRABARTI, D.: "Further evidence on the PET cyclization of alpha-silylmethylamines tethered with non-activated olefins:demonstration by the total synthesis of (-)-retronecanol" TETRAHEDRON LETTERS, vol. 37, no. 13, 1996, pages 2285-2288, XP004842006
- TRYBULSKI, EUGENE J. ET AL: "Synthesis and structure activity relationships of enantiomerically pure hydroxylated oxotremorine derivatives" BIOORGANIC & MEDICINAL CHEMISTRY LETTERS , 2(8), 827-32 CODEN: BMCLE8; ISSN: 0960-894X, 1992, XP009050461

## Description

The present invention relates to a process for preparing an amine which is useful as an intermediate for synthesis of e.g. medicines and agrochemicals.

Processes for preparing an amine by decarboxylating an α-amino acid have been known for long time, but there are many methods which have problems not desirable for industrial application such as requiring drastic heating for long term, or a catalyst such as a dangerous peroxide.

Recently methods which solve the above problems and may be useful for industrial application have been reported.

For example, (1) an amine is prepared by decarboxylating an α-amino acid under heating in the presence of a vinyl ketone as catalyst in a solvent such as cyclohexanol (JP-B-4-10452), (2) an amine is prepared by decarboxylating an α-amino acid under heating in the presence of a monoaryl ketone as catalyst in a solvent such as cyclohexanol (JP-A-5-255204), (3) an amine is prepared by decarboxylating cis-3-hydroxy-L-proline or trans-3-hydroxy-L-proline in the absence of catalyst in a solvent such as cyclohexanol (WO97/43256), and (4) an amine is prepared by heating an α-amino acid and an aliphatic saturated ketone followed by hydrogenation (JP-A-2001-220372).

The preparation of (R)-3-hydroxypyrrolidine from L-hydroxyproline by decarboxylation under heating with a catalytic amount of 2-cyclohexen-1-one in tetraethylene glycol dimethylether is disclosed in:
MEHLER, T. ET AL: "Enantioselective addition of diethylzinc to aromatic aldehydes catalyzed by chiral ligands derived from L-hydroxyproline" SYNTHETIC COMMUNICATIONS, 23(19), 2691-9 CODEN: SYNCAV; ISSN: 0039-7911, 1993, XP009033906;
HASHIMOTO, M.; EDA, Y.; OSANAI, Y. ET AL.: "A novel decarboxylation of alpha-amino acids. A facile method of decarboxylation by the use of 2-cyclohexen-1-one as a catalyst" CHEMISTRY LETTERS, 1986, pages 893-896, XP000891448; and
WALLBAUM, S.: MEHLER, T. ET AL.: "Decarboxylation of alpha-amino acids containing two and three stereogenic centers: a simple one-step procedure to prepare two optically active beta-amino alcohols and bicyclic pyrrolidine derivative", SYNTHETIC COMMUNICATIONS, 24(10), 131-1387 (1994).

By the way, many of the amines prepared by decarboxylating α-amino acids are unstable when heated. For example, as regards 3-hydroxypyrrolidine prepared by decarboxylating 4-hydroxyproline, the decrease of its weight begins at around 50°C and drastic thermolysis is observed at around 120°C according to its thermogravimeteric analysis (TG/DTA). As can be seen from the thermoanalysis, it is difficult to isolate the unstable product prepared by decarboxylating an α-amino acid by the usual procedure such as concentration of the reaction mixture followed by distillation.

Therefore, a safe and reproducible isolation method of amines prepared by decarboxylating an α-amino acid has been desired. However, the following problems have not yet been solved by above mentioned methods.

Namely, as regards methods (1) to (3), isolation and purification is carried out by converting the object compound into its hydrochloride and crystallization after decarboxylating, The steps are complex and troublesome, and isolation and purification of the object compound as free amine has not been established. As regards method (4), isolation and purification of the object compound as free amine is carried out by adding water to the reaction mixture, extracting the object compound, concentrating the water layer and then distilling in vacuo, but it is not said that this conventional procedure solves the problem of the above mentioned thermolysis.

An object of the invention is to provide a process to isolate amines safely and reproducibly.

This object could be achieved by a safe and reproducible method for isolating amines sensitive to thermolysis prepared by decarboxylating an α-amino acid in the form of free amines.

Namely, the present invention relates to a process for preparing an amine which comprises preparing an amine by decarboxylating an α-amino acid under heating in the presence of a vinyl ketone as a catalyst in a poly C_{1 to 6} alkylene glycol having an average molecular weight of 200 to 6000 as a solvent, and directly recovering said amine by distillation from the same reaction system, namely in one pot.

As the present invention involves the use of a poly C_{1 to 6} alkylene glycol as a solvent and it is not necessary to remove the solvent, it becomes simple to recover the object compound by distillation and the stability of the object compound is promoted even under heating.

The α-amino acid used in this process is not limited as far as the amino group and the carboxyl group on the compound are present on the same carbon atom, and the amino group has at least one hydrogen atom.

For example, an α-amino acid having an alkyl group or aryl group as a side chain such as alanine, leucine, isoleucine, valine, phenylglycine, phenylalanine and tyrosine, a cyclic amino acid such as proline, hydroxyproline and pipecolic acid, an α-amino acid having a functional group like a carboxyl group, amino group, hydroxy group, mercapto group or methyl mercapto group such as aspargic acid, glutamic acid, lysine, omithine, threonine, methionine, cysteine and triptophan, and furthermore, a derivative thereof and an α-amino acid having other substituent are illustrated.

Therefore, when the α-amino acid has a hydroxy group on a carbon atom other than the carbon atom at α position, the amine having a hydroxy group on said carbon atom is obtained by the decarboxylating reaction. For example, when the α-amino acid is 4-hydroxyproline, 3-hydroxypyrrolidine is obtained as an amine. Especially, when said carbon atom in the α-amino acid is asymmetric, the optically active corresponding amine is obtained by the decarboxylating reaction.

Among the poly C_{1 to 6} alkylene glycol, used in this reaction polyethylene glycol and polypropylene glycol are especially preferable.

The decarboxylating reaction is carried out at 120°C to 250°C, preferably at 140°C to 170°C. The reaction is carried out at atmospheric pressure, but may be carried out under pressure. The reaction period is suitably selected depending on the reaction temperature and the reaction pressure.

A vinyl ketone such as 2-cyclopenten-1-one, 2-cyclohexen-1-one and 2-cyclohepten-1-one is used as the catalyst. The amount is preferably 0.01 to 0.2moles to the α-amino acid.

After the reaction, as the boiling point of the poly C_{1 to 6} alkylene glycol used as a solvent is higher than that of the produced amine, the conventional removal procedure of the solvent before distillation of the product is not required and therefore, the object amine in free form is obtainable by directly distillating the reaction mixture under reduced pressure in one pot.

In this reaction, the poly C_{1 to 6} alkylene glycol serves not only as a solvent, but also as a stabilizer during distillation at high temperature and, therefore during the distillation procedure the amine is obtained without remarkable degradation.

The present invention is in detail explained by the following examples but the present invention should be limited by these examples.

The measurement of optical purity was conducted by HPLC (CHIRALCEL AS: Daicel Chemical) by benzoylation of the hydroxy group of 3-pyrrolidinol.

### Example 1

### (R)-3-Pyrrolidinol

To a 1 liter reaction vessel were added (4R)-hydroxy-L-proline (250g, 1.907mol), 2-cyclohexen-1-one (18.3g, 0.1907mol) and polyethylene glycol (750 ml; average molar weight 400), and the mixture was stirred under a stream of nitrogen gas at 150~160°C. After 10 hours, when the crystals were disappeared and the mixture became homogenous, the reaction was judged to be completed. By quantitatively analyzing by gas chromatography, the yield of (R)-3-pyrrolidinol was 89.3%. Then at the same temperature, the reaction solution was directly distilled under reduced pressure to give (R)-3-pyrrolidinol as a distilled portion of 100~120°C (6~40hPa) (120.4g, yield: 72.5%, chemical purity: 99.6%, optical purity: 99.9%ee).

### Example 2

### (R)-1-Amino-2-propanol

To a 500ml reaction vessel were added L-threonine (100g, 0.839mol), 2-cyclohexen-1-one (8.07g, 0.084mol) and polyethylene glycol (300 ml, average molar weight 400), and the mixture was stirred under a stream of nitrogen gas at 150~160°C. After 72 hours, when the crystals were disappeared and the mixture became homogenous, the reaction was judged to be completed. By quantitatively analyzing by gas chromatography, the yield of (R)-1-amino-2-propanol was 80.6%. Then at the same temperature, the reaction solution was directly distilled under reduced pressure to give (R)-1-amino-2-propanol as a distilled portion of 54~62°C (1~20 hPa) (44.4g, yield: 70.4%, chemical purity: 99.0%, optical purity: 99.9%ee).

### Example 3

To an amino acid (0.100mol) selected from leucine, isoleucine, valine, phenylglycine, lysine, methionine and triptophan, are added 2-cyclohexen-1-one (0.100mol) and polyethylene glycol (three times as much as the amino acid). The mixture is stirred under a stream of nitrogen gas at 150~160°C. When the crystals are disappeared and the mixture becomes homogenous, the reaction is judged to be completed. Then at the same temperature, the reaction solution is directly distilled under reduced pressure. There is obtainable a corresponding amine.

### Comparative example

To a 300ml reaction vessel were added (4R)-hydroxy-L-proline (75.0g, 0.572mol), 2-cyclohexen-1-one (5.50g, 57.20mmol) and cyclohexanol (225ml), and the mixture was stirred under a stream of nitrogen gas at 15Q~160°C. After 8 hours, when the crystals were disappeared and the mixture became homogenous, the reaction was judged to be completed. By quantitatively analyzing by gas chromatography, the yield of (R)-3-pyrrolidinol was 83.3%. Then at 70-90°C on a water bath, the reaction solution was concentrated under reduced pressure and the residue was analyzed by gas chromatography. As a result the yield of (R)-3-pyrrolidinol was 68.8% and the degradative ratio by concentration procedure was 14.5%. The residue was further distilled as a distilled portion of 100~116°C (4 hPa) to give (R)-3-pyrrolidinol (28.75g, isolation yield: 57.7%, chemical purity: 93.9%, optical purity: 99.9%ee).

The present invention is utilized for preparing amines which are useful as intermediate for synthesis of a variety of medicines such as antibacterials and bronchodilators, agrochemicals and physiologically active compounds.

## Claims

1. A process for preparing an amine which comprises preparing an amine by decarboxylating an α-amino acid under heating at 120 to 250°C in the presence of a vinyl ketone as a catalyst in a poly C_{1 to 6} alkylene glycol having an average molecular weight of 200 to 6000 as a solvent, and directly recovering said amine by distillation in the same reaction system.

2. The process according to claim 1 wherein the poly C_{1 to 6} alkylene glycol is polyethylene glycol or polypropylene glycol.

3. The process according to claim 1 or 2 wherein the α-amino acid has a hydroxy group on a carbon atom other than the carbon atom at the α-position, and the amine has a hydroxy group.

4. The process according to any one or claims 1 to 3 wherein the α-amino acid is 4-hydroxyproline, and the amine is 3-hydroxypyrrolidine.

5. The process according to any one of claims 1 to 4 wherein the α-amino acid has an asymmetric carbon atom at another position than at the α-position of said α-amino acid, and the amine is an optically active amine.

## Patentansprüche

1. Verfahren zur Herstellung eines Amins, welches das Herstellen eines Amins durch Decarboxylierung einer α-Aminosäure unter Erwärmung bei 120 bis 250°C in Gegenwart eines Vinylketons als einen Katalysator in einem Poly-C₁₋₆-alkylenglycol mit einem durchschnittlichen Molekulargewicht von 200 bis 6000 als ein Lösungsmittel und das direkte Gewinnen des Amins durch Destillation im gleichen Reaktionssystem umfasst.

2. Verfahren gemäß Anspruch 1, wobei das Poly-C₁₋₆-alkylenglycol Polyethylenglycol oder Polypropylenglycol ist.

3. Verfahren gemäß Anspruch 1 oder 2, wobei die α-Aminosäure eine Hydroxylgruppe an einem anderen Kohlenstoffatom als dem Kohlenstoffatom in der α-Position aufweist und das Amin eine Hydroxylgruppe aufweist.

4. Verfahren gemäß einem der Ansprüche 1 bis 3, wobei die α-Aminosäure 4-Hydroxyprolin ist und das Amin 3-Hydroxypyrrolidin ist.

5. Verfahren gemäß einem der Ansprüche 1 bis 4, wobei die α-Aminosäure ein asymmetrisches Kohlenstoffatom an einer anderen Position als der α-Position der α-Aminosäure aufweist und das Amin ein optisch aktives Amin ist.

## Revendications

1. Procédé de préparation d'une amine qui comprend la préparation d'une amine par décarboxylation d'un α-aminoacide en chauffant de 120 à 250°C en présence d'une vinylcétone comme catalyseur dans un polyalkylèneglycol en C₁-C₆ présentant un poids moléculaire moyen de 200 à 6 000 comme solvant et par récupération directe de ladite amine par distillation dans le même système réactionnel.

2. Procédé selon la revendication 1, dans lequel le polyalkylèneglycol en C₁-C₆ est le polyéthylèneglycol ou le polypropylèneglycol.

3. Procédé selon la revendication 1 ou 2, dans lequel l'α-aminoacide présente un groupe hydroxy sur un atome de carbone différent de l'atome de carbone à la position α et l'amine présente un groupe hydroxy.

4. Procédé selon l'une quelconque des revendications 1 à 3, dans lequel l'α-aminoacide est la 4-hydroxyproline et l'amine est la 3-hydroxypyrrolidine.

5. Procédé selon l'une quelconque des revendications 1 à 4, dans lequel l'α-aminoacide présente un atome de carbone asymétrique à une autre position que la position α dudit α-aminoacide et l'amine est une amine optiquement active.
